# EUROPÄISCHE PATENTSCHRIFT

(11) **EP 2 281 029 B1**
(45) Veröffentlichungstag und Bekanntmachung des Hinweises auf die Patenterteilung: **20.06.2012**
(21) Anmeldenummer: 09734981.5
(22) Anmeldetag: 24.04.2009
(51) Int. Cl.: C12M 3/08

(54) **VERFAHREN UND VORRICHTUNG ZUM AUFSCHLUSS VON BIOLOGISCHEM MATERIAL**
METHOD AND DEVICE FOR BREAKING DOWN BIOLOGICAL MATERIAL
PROCÉDÉ ET DISPOSITIF DE DÉCOMPOSITION D'UN MATÉRIAU BIOLOGIQUE

(30) Priorität: 25.04.2008 DE 102008021000
(43) Veröffentlichungstag der Anmeldung: 09.02.2011
(73) Patentinhaber: Qiagen GmbH, 40724 Hilden (DE)
(72) Erfinder: ROTHMANN, Thomas, 40764 Langenfeld (DE); SCHMID, Claudia, 45130 Essen (DE); SCHÄFER, Andreas, 51375 Leverkusen-Schlebusch (DE); CHRISTOFFEL, Gabriele, 51061 Köln (DE)
(86) Internationale Anmeldenummer: PCT/EP2009/054948
(87) Internationale Veröffentlichungsnummer: WO 2009/130300

(56) Entgegenhaltungen:
- EP-A- 0 353 365
- EP-A- 1 664 264
- CN-A- 101 233 910
- US-A1- 2001 012 612
- US-A1- 2002 039 783
- US-B1- 6 719 449

## Beschreibung

Die Erfindung betrifft eine Vorrichtung zum Aufschluss von biologischem Material, insbesondere zur Gewinnung von Biomolekülen, mittels Ultraschall, wobei das biologische Material in einem Behältnis zusammen mit einer Flüssigkeit angeordnet wird, sowie ein mit dieser Vorrichtung durchführbares Verfahren.

Derartige Verfahren, wie beispielsweise die Verwendung von Ultraschall zum Aufschluss von Materialien, wie beispielsweise organisches oder anorganisches Probenmaterial oder fossiles Material, sind aus dem Stand der Technik bekannt. Der Aufschluss erfolgt hierbei, um Zielbestandteile des aufzuschließenden Materials zu erhalten. Beim Aufschließen wird zwischen offenen und geschlossenen Umgebungsparametern unterschieden. Wobei das Aufschließen in einem geschlossenen Gefäß (siehe beispielsweise US-A-6100084) durchaus relevant sein kann, da damit ein verunreinigungsfreier Aufschluss erreicht werden kann, der insbesondere für das Aufschließen von biologischem Material, von Vorteil ist. Das aufzuschließende Material befindet sich hierbei in dem Gefäß in einer Flüssigkeit und wird mit Ultraschall beaufschlagt.

Nachteilig beim Aufschluss mittels Ultraschall in einem geschlossenen Gefäß ist, dass das Aufschlussmaterial in der Flüssigkeit frei beweglich ist. Damit befindet sich das Material entweder in einem Fokuspunkt des Ultraschalls und wird aufgeschlossen oder nicht. Weiterhin ist der Grad des Aufschlusses sehr schwierig zu beeinflussen.

US-A-6878540, US-A-6881541, US-A-6887693 und US-A-20060019379 offenbaren ebenfalls den Einsatz von Ultraschall zum Aufschluss von Material. Als ein Transducer wird ein Ultraschalltrichter verwendet, der direkt an die Wand des Containers angedockt ist. Zusätzlich werden der Flüssigkeit im Gefäß Kügelchen zugegeben, die einen besseren Aufschluss des Materials bewirken sollen. Weiterhin wird offenbart, das Gefäß, das einen flexiblen Wandabschnitt aufweist, unter Druck zu setzen, um die Verbindung zwischen Gefäß und Transducer zu verbessern und damit den Übergang der Schallwellen in das Gefäß zu optimieren. Die beschriebenen Verfahren sind jedoch aufwendig, kostenintensiv und führen nur bedingt zum Erfolg.

US-B-6719449 offenbart ebenfalls eine Vorrichtung und ein Verfahren zum Aufschluss von Proben mittels einer berührungslosen Behandlung durch Ultraschall, bei dem ein fokussierter Energiestrahl verwendet wird. Dabei werden die Größen des Probenbehälters so gewählt, dass der gesamte Inhalt sich im Fokuspunkt befindet. Dadurch sind lediglich Aufschlüsse von geringen Materialmengen möglich. Ferner muss die Beaufschlagungszeit der Probe überwacht werden, um keine Überschallung zu erzeugen.

Die EP 0 353 365 A2 beschreibt eine Vorrichtung zum Aufschluss von biologischen Material, welches in Aufnahmebehältern befindlich ist, welche in einem Bad, das mit Ultraschallwellen beaufschlagbar ist, aufgenommen sind. Die Behälter werden in diesem Bad langsam rotatorisch bewegt und die Probe ist im Behälterinneren frei beweglich. Um die Ultraschallwellen in einem Punkt im Inneren des Behälters zu fokussieren, ist an den Behältern jeweils eine Nut vorgesehen. Dies ist jedoch nachteilig, da keine Standardbehälter Verwendung finden können. Darüber hinaus kann nicht gewährleistet werden, dass sich das gesamte aufzuschließende Material während des Vorgangs im unteren Bereich des Behälters befindet, an dem auch der Fokuspunkt der Ultraschallwellen angeordnet ist.

Der Erfindung liegt die Aufgabe zugrunde, die zuvor genannten Nachteile weitestgehend zu überwinden und einen definierten Aufschluss des Aufschlussmaterials zu bewirken.

Gelöst wird diese Aufgabe hinsichtlich der Vorrichtung dadurch, dass eine Aufnahme für ein Behältnis vorgesehen ist, weiterhin eine Rotationsvorrichtung zum Ausüben einer Zentrifugalkraft auf das Behältnis und ein Ultraschallemitter, der im Bereich des Behältnisses in Wirkrichtung der Zentrifugalkraft angeordnet ist. Während des Zentrifugierens sind Ultraschallwellen erzeugbar, die in einem Punkt fokussierbar sind, der identisch mit dem Konzentrationspunkt der Zentrifugalkraft ist, wobei die im Fokus entstehende, durch den Ultraschall hervorgerufene Kraft entgegengesetzt zur Zentrifugalkraft wirkt. Somit liegt das aufzuschließende Material aufgrund der Zentrifugalkraft in einem vorgegebenen Punkt im Behältnis konzentriert vor, der mit dem Konzentrationspunkt im Wesentlichen übereinstimmt. Bei dem aufzuschließenden Material kann es sich dabei um organisches und/oder anorganisches Probenmaterial, wie beispielsweise fossiles oder biologisches Material handeln. Ferner kann das Material einstückig vorliegen, beispielsweise in Form von Gewebematerial, oder aus mehreren, einzelnen Bestandteilen bestehen. Vorzugsweise handelt es sich bei dem aufzuschließenden Material um biologisches Material, wie Gewebe, Zellen, Sporen etc., welches sich insbesondere zur Gewinnung von Biomolekülen, wie Zellbestandteile, Proteine, Lipide, Kohlenhydrate, Nukleinsäuren etc., eignet.

Weiterhin wird die der Erfindung zugrunde liegende Aufgabe durch ein Verfahren gelöst, welches sich dadurch auszeichnet, dass das biologische Material im Behältnis frei beweglich ist, dass das biologische Material mittels Zentrifugalkraft zu einem vorbestimmten Punkt des Behältnisses bewegt wird und dass während des Zentrifugierens Ultraschallwellen erzeugt werden, wobei die Ultraschallwellen in einem Punkt fokussiert werden, der identisch mit dem Konzentrationspunkt ist, wobei die im Fokus entstehende, durch den Ultraschall hervorgerufene Kraft entgegengesetzt zur Zentrifugalkraft wirkt.

Da sowohl die Ultraschallkraft als auch die Zentrifugalkraft so eingestellt sind, dass sie entgegenwirkend hinsichtlich der Wirkrichtung sind, bewirkt dieses, dass nach einem ausreichenden Aufschluss des Materials, der, auf die kleineren, nicht löslichen, Partikel des aufzuschließenden Materials, die sich im Fokuspunkt befinden, wirkende Kraftanteil der Zentrifugalkraft abnimmt, und diese Teile aus dem Fokuspunkt wandern, und damit die Wirkung der Ultraschallkraft auf diese Partikel erst reduziert und dann vollständig beendet wird. So wird ferner vorteilhafterweise mit dem Aufschließen gleichzeitig nicht nur das direkt in Lösung gegangene aufgeschlossene Material (vorzugsweise die zu gewinnenden Biomoleküle) sondern auch das nicht lösliche bzw. das nicht direkt in die Lösung übergegangene, aufgeschlossene Material von dem nicht aufgeschlossenem Material getrennt, wodurch insbesondere die notwendige Zeit reduziert wird, um den vollständigen Aufschluss herbeizuführen. Bei dem aufgeschlossenen Material handelt es sich vorzugsweise um kleine lösliche und/oder unlösliche Aufschlussbestandteile, besonders bevorzugt um Biomoleküle.

Das nicht aufgeschlossene bzw. nicht aufschließbare Material (bei biologischem Material beispielsweise der Zelldebris etc.) bildet dabei durch die Zentrifugalkraft vorteilhafterweise beeinflusst ein Pellet und verbleibt in diesem.

Eine vorteilhafte Lehre der Erfindung sieht vor, dass die Stärke der Zentrifugalkraft in Abhängigkeit der Partikelgröße des zum Aufschluss bestimmten biologischen Materials eingestellt wird. Auf dieses Weise ist es möglich, definierte Aufschlussprofile in Abhängigkeit des aufzuschließenden Materials und des gewünschten Aufschlussgrades einzustellen.

Eine weitere vorteilhafte Lehre der Erfindung sieht vor, dass die Ultraschallwellen mittels eines Ultraschallemitters erzeugt werden, der bevorzugt direkt im Bereich des Fokuses des vorbestimmten Punktes des Behältnisses angeordnet ist. Dadurch kann eine direkte Abgabe erfolgen. Weiterhin wird die Verbindung zwischen Emitter und Behältnis durch die Zentrifugalkraft verbessert.

Eine weiterhin vorteilhafte Ausführung des erfindungsgemäßen Verfahrens ist, dass das Behältnis und/oder die Flüssigkeit gekühlt werden. Dadurch wird bei bestimmten Aufschlussmaterialien, insbesondere bei biologischen Materialien, eine Veränderung des Materials aufgrund der durch den Ultraschall erzeugten Wärme verhindert.

Die erfindungsgemäße Vorrichtung bewirkt auf einfache Weise die maschinelle Umsetzung des erfindungsgemäßen Verfahrens.

Eine weitere vorteilhafte Lehre der Erfindung sieht vor, dass der Ultraschallemitter in Richtung der Zentrifugalkraft angeordnet ist. Dadurch kann der wirkenden Zentrifugalkraft auf einfache Weise die Ultraschallkraft entgegengerichtet werden.

Nachfolgend wird die Erfindung anhand eines Ausführungsbeispiels anhand einer Zeichnung näher erläutert, wobei:
- Fig. 1: Seitenansicht eines Behältnisses mit Flüssigkeit und Aufschlussmaterial,
- Fig. 2: Seitenansicht zu Fig. 1 während des Zentrifugierens,
- Fig. 3: Seitenansicht zu Fig. 2 mit Ultraschallbeaufschlagung,
- Fig. 4: Seitenansicht zu Fig. 1 mit aufgeschlossenem Material, und
- Fig. 5: Seitenansicht zu Fig.4 während der Entnahme des aufgeschlossenen Materials.

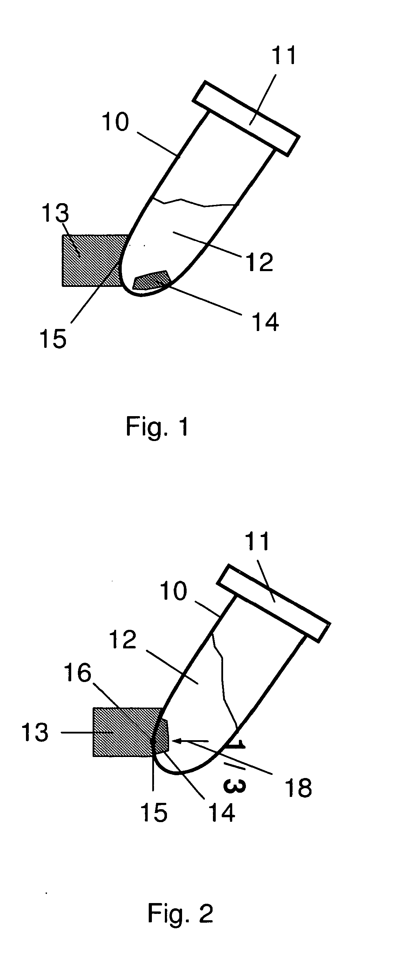

Fig. 1 bis Fig. 5 zeigen den Ablauf des erfindungsgemäßen Verfahrens.

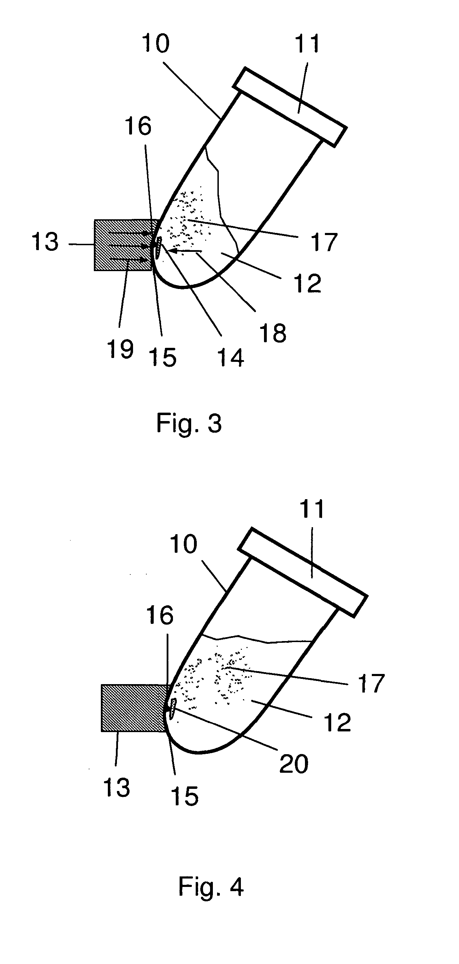

In Fig. 1 ist ein Behältnis 10 dargestellt, das mit einem Deckel 11 verschlossen ist. In dem Behältnis 10 befindet sich eine Flüssigkeit 12 sowie darin ein aufzuschließendes Material 14. An einem Wandabschnitt 15 des Behältnisses 10 ist eine Utraschallemitter 13 angeordnet. Das Behältnis 10 befindet sich in einer Vorrichtung (nicht dargestellt), mit der eine Zentrifugalkraft 18 erzeugt wird, die nach außen gerichtet ist, wie in Fig. 2 und 3 dargestellt ist. Die Zentrifugalkraft 18 bewirkt, dass das aufzuschließende Material 14 sich in einem Fokuspunkt 16 an dem Wandabschnitt 15 anordnet. In dem Fokuspunkt 16 bündelt sich das aufzuschließende Material 14. Weiterhin bündeln sich hier auch die von dem Emitter 13 erzeugten Ultraschallwellen, siehe Fig. 3, so dass hier eine Kraftentfalltung der Ultraschallkraft 19 auftritt, wodurch das aufzuschließende Material 14 aufgeschlossen wird. Gleichzeitig werden die nicht aufschließbaren bzw. unlöslichen Bestandteile des aufzuschießenden Materials 14, bedingt durch die Zentrifugalkraft, zu einem Pellet 20 geformt, welches sich in der Flüssigkeit 12 am Rand des Behältnisses 10, vornehmlich im Bereich des Wandabschnitts 15 absetzt resp. verbleibt.

Fig. 4 zeigt das Behältnis 10 nach Durchführung des Aufschlusses. Das aufgeschlossene Material 17, welches in Lösung gegangen ist oder aufgrund der Gewichtsabnahme der einzelnen Partikel nicht mehr im Fokuspunkt 16 gehalten wurde, befindet sich in gelöster und/oder partikulärer Form in der Flüssigkeit 12. An dem Wandabschnitt 15 bzw. in seiner Nähe befinden sich das Pellet 20.

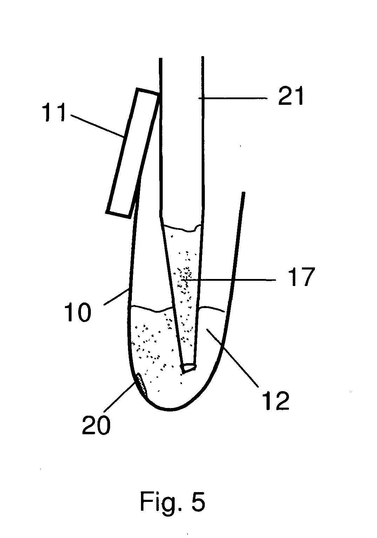

Fig. 5 zeigt die Entnahme des aufgeschlossenen Materials 17 aus dem Behälter 10 mittels einer Entnahmevorrichtung 21, wie beispielsweise einer Pipette.

### Bezugszeichenliste

- 10: Behältnis
- 11: Deckel
- 12: Flüssigkeit
- 13: Emitter
- 14: aufzuschließendes Material
- 15: Wandabschnitt
- 16: Fokuspunkt
- 17: aufgeschlossenes Material
- 18: Zentrifugalkraft
- 19: Ultraschallkraft
- 20: Pellet der nicht aufschließbaren Bestandteile
- 21: Entnahmevorrichtung

## Patentansprüche

1. Vorrichtung mit einer Aufnahme für ein Behältnis (10) mit einer Rotationsvorrichtung zum Ausüben einer Zentrifugalkraft auf das Behältnis und einem Ultraschallemitter (13), der im Bereich des Behältnisses in Wirkrichtung der Zentrifugalkraft dem Fokuspunkt (16) angeordnet ist, wobei während des Zentrifugierens Ultraschallwellen erzeugbar sind, die in einem Punkt fokussierbar sind, der identisch mit dem Konzentrationspunkt der Zentrifugalkraft ist, wobei die im Fokus entstehende, durch den Ultraschall hervorgerufene Kraft entgegengesetzt zur Zentrifugalkraft wirkt.

2. Verfahren zum Aufschluss von biologischem Material, insbesondere zur Gewinnung von Biomolekülen, mittels Ultraschall wobei das biologische Material in einem Behältnis zusammen mit einer Flüssigkeit angeordnet wird und wobei eine Vorrichtung gemäß Anspruch 1 Verwendung findet.

3. Verfahren nach Anspruch 2, **dadurch gekennzeichnet, dass** die Stärke der Zentrifugalkraft in Abhängigkeit der Partikelgröße des zum Aufschluss bestimmten biologischen Materials eingestellt wird.

4. Verfahren nach Anspruch 2 oder 3, **dadurch gekennzeichnet, dass** die Ultraschallwellen mittels eines Ultraschallemitters erzeugt werden, der bevorzugt direkt im Bereich des Fokuses des vorbestimmten Punktes des Behältnisses angeordnet ist.

5. Verfahren nach einem der Ansprüche 2 bis 4, **dadurch gekennzeichnet, dass** das Behältnis und/oder die Flüssigkeit gekühlt werden.

## Claims

1. Device having a receptacle for a container (10), a rotation device for exerting a centrifugal force on the container, and an ultrasonic emitter (13) that is arranged in the region of the container in the direction of action of the centrifugal force at the focal point (16), ultrasonic waves being able to be generated during the centrifugation, which ultrasonic waves are able to be focused at a point that is identical to the concentration point of the centrifugal force, the force resulting at the focus and caused by the ultrasound acting in opposition to the centrifugal force.

2. Method for breaking down biological material, in particular for obtaining biomolecules, by means of ultrasound, the biological material being arranged in a container together with a liquid and a device according to Claim 1 being used.

3. Method according to Claim 2, **characterized in that** the strength of the centrifugal force is set as a function of the particle size of the biological material intended to be broken down.

4. Method according to Claim 2 or 3, **characterized in that** the ultrasonic waves are produced by means of an ultrasonic emitter which preferably is arranged directly in the region of the focus of the predetermined point of the container.

5. Method according to one of Claims 2 to 4, **characterized in that** the container and/or the liquid are/is cooled.

## Revendications

1. Dispositif comprenant un réceptacle destiné à un récipient (10) comportant un dispositif de mise en rotation destiné à exercer une force centrifuge sur le récipient et un émetteur d'ultrasons (13) qui est disposé dans la région du récipient dans la direction d'action de la force centrifuge au foyer, dans lequel, pendant la centrifugation, il est possible de générer des ondes ultrasonores qui peuvent être focalisées en un point identique au point de concentration de la force centrifuge, dans lequel la force apparaissant au foyer et créée par les ultrasons est opposée à la force centrifuge.

2. Procédé de décomposition de matière biologique, notamment destiné à récupérer des biomolécules au moyen d'ultrasons, dans lequel la matière biologique est disposée dans un récipient en association avec un liquide et dans lequel un dispositif selon la revendication 1 est utilisé.

3. Procédé selon la revendication 2, **caractérisé en ce que** l'intensité de la force centrifuge est réglée en fonction de la taille de particule de la matière biologique déterminée pour la décomposition.

4. Procédé selon la revendication 2 ou 3, **caractérisé en ce que** les ondes ultrasonores sont générées au moyen d'un émetteur à ultrasons qui est de préférence disposé directement dans la région du foyer du point prédéterminé du récipient.

5. Procédé selon l'une quelconque des revendications 2 à 4, **caractérisé en ce que** le récipient et/ou le liquide sont refroidis.
